(19) Europäisches Patentamt
European Patent Office
Office européen des brevets

(11) Numéro de publication : **0 107 536**
**B1**

(12) # FASCICULE DE BREVET EUROPÉEN

(45) Date de publication du fascicule du brevet : **28.05.86**

(21) Numéro de dépôt : **83401829.3**

(22) Date de dépôt : **20.09.83**

(51) Int. Cl.[4] : **C 07 D253/08**, C 07 D495/04, A 61 K 31/53// C07D295/08, C07D295/06 ,(C07D495/04, 333:00, 253:00)

(54) **Benzo- et thiéno-triazine-1,2,3 ones-4, procédés de préparation et médicaments les contenant.**

(30) Priorité : **22.09.82 FR 8215934**

(43) Date de publication de la demande : **02.05.84 Bulletin 84/18**

(45) Mention de la délivrance du brevet : **28.05.86 Bulletin 86/22**

(84) Etats contractants désignés : **AT BE CH DE FR GB IT LI LU NL SE**

(56) Documents cités :
**DE-A- 1 926 076**
**FR-A- 1 578 785**
**US-A- 3 316 262**
**US-A- 3 808 318**

(73) Titulaire : **LIPHA, LYONNAISE INDUSTRIELLE PHARMACEUTIQUE**
**34, rue Saint Romain Boîte Postale 8481**
**F-69359 Lyon Cedex 08 (FR)**

(72) Inventeur : **Ferrand, Gérard**
**62, rue des Aqueducs**
**F-69005 Lyon (FR)**
Inventeur : **Dumas, Hervé**
**105, avenue du Vellein**
**F-38290 Villefontaine (FR)**
Inventeur : **Bayssat, Michel**
**Chemin de Beckensteiner**
**F-69260 Charbonnieres (FR)**
Inventeur : **Depin, Jean-Claude**
**113, Cours Gambetta**
**F-69003 Lyon (FR)**

(74) Mandataire : **Bouton Neuvy, Liliane et al**
**L'Air liquide, Société Anonyme pour L'Etude et L'Exploitation des Procédés Georges Claude 75, Quai d'Orsay**
**F-75321 Paris Cedex 07 (FR)**

**EP 0 107 536 B1**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

**Description**

La présente invention concerne des [(phényl-4 pipérazinyl-1)-3 alcoyl]-3-3H-benzo-et thiéno-triazine-1,2,3 ones-4, des procédés permettant de les préparer et leur application dans le domaine thérapeutique.

Le rôle possible de l'hydroxy-5 tryptamine (5-HT) dans l'étiologie de la dépression suscite un grand intérêt dans la recherche d'inhibiteurs sélectifs de sa recapture neuronale. On a découvert des composés présentant cette propriété et pouvant être utilisés dans le traitement des états dépressifs et des troubles psychiques.

On connaît la dichloro-6,8 (phényl-4 pipérazinométhyl)-3-3H-benzotriazine-1,2,3 one-4 par les travaux décrits dans le brevet français 1 578 785 de N.V. Philips'Gloeilampenfabrieken, comme dépresseur du système nerveux central. A. Stachel et R. Beyerle décrivent dans le brevet allemand 1 926 076 une [hydroxy-2 (phényl-4 pipérazinyl-1)-3 propyl]-3 triméthoxy-6,7,8-3H-benzotriazine-1,2,3 one-4 parmi une série de (γ-amino β-hydroxypropyl)-3-3H-benzotriazine-1,2,3 ones-4 servant d'intermédiaires à des produits cardiovasculaires.

Les composés objets de l'invention sont représentés par la formule générale I.

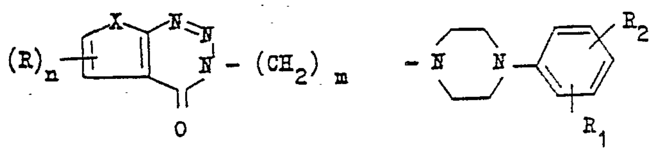

(I)

dans laquelle m est 2, 3 ou 4, X est le groupe vinylène —CH = CH— ou un atome de soufre. Lorsque X = —CH = CH—, R peut occuper une position quelconque sur le noyau aromatique et peut être l'hydrogène, un halogène, un radical alcoyle inférieur, alcoxy inférieur ou le groupe nitro, n étant 0, 1, 2 ; lorsque X est un atome de soufre, R est l'hydrogène ou R constitue une chaîne $-(CH_2)_4-$ entre les deux positions libres du cycle thiophénique. $R_1$ et $R_2$ peuvent être égaux ou différents et sont l'hydrogène, un halogène, un radical alcoyle inférieur, alcoxy inférieur ou trifluorométhyle. $R_1$ et $R_2$ peuvent occuper n'importe quelle position sur le noyau aromatique.

Le terme inférieur appliqué à un groupe alcoyle ou alcoxy signifie que le groupe peut être linéaire ou ramifié et qu'il peut comprendre de 1 à 3 atomes de carbone.

Les composés dans la formule desquels $R_1$ et $R_2$ sont l'hydrogène ou un halogène constituent une classe particulièrement intéressante.

Les sels pharmaceutiquement acceptables font partie intégrante de l'invention. Ce peuvent être des sels préparés soit à partir d'acides minéraux comme l'acide chlorhydrique, l'acide bromhydrique, l'acide sulfurique, l'acide phosphorique, soit à partir d'acides organiques comme l'acide tartrique, l'acide citrique, l'acide acétique, l'acide maléique, l'acide fumarique, l'acide oxalique, l'acide méthanesulfonique.

Les composés de l'invention peuvent se préparer selon l'une au moins des méthodes suivantes :

a) Une triazine-1,2,3 one-4 de formule II

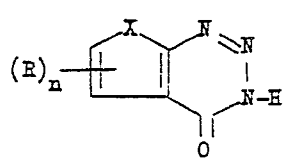

(II)

est alcoylée par un dérivé de formule III

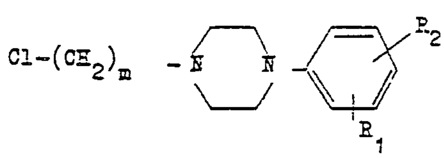

(III)

Dans les formules II et III, X, R, $R_1$, $R_2$, m et n ont les significations données précédemment. La réaction s'effectue dans un solvant inerte en présence d'un agent alcalin. Les solvants préférés sont les alcanols de bas poids moléculaire, et surtout l'acétonitrile et le N,N-diméthylformamide. Les bases utilisées peuvent être des alcoolates, des hydroxydes ou des carbonates alcalins. La température peut varier entre la température ambiante et la température d'ébullition du solvant utilisé. Le temps de réaction est généralement compris entre 1 et 12 heures.

Les triazine-1,2,3 ones-4 de formule II constituent une classe de composés connue et plusieurs de

celles utilisées ont déjà été décrites. Celles qui sont nouvelles ont été préparées selon des techniques usuelles, par diazotation soit des amides soit des esters méthyliques ou éthyliques des acides anthraniliques correspondants — par exemple A. Weddige et H. Finger J. Prakt ; Chem. *35*, 262 (1882). De même, plusieurs des dérivés de formule III utilisés sont connus. Les autres ont été préparés par alcoylation d'une phényl-1 pipérazine convenablement substituée par le bromo-1 ω-chloroalcane selon les techniques décrites par exemple par J. Bourdais [Bull. Soc. Chim. France 3246 (1968)] ou par C.B. Pollard et coll. [J. Org. Chem. *24*, 764 (1959)].

b) Une benzotriazine-1,2,3 one-4 de formule générale IV

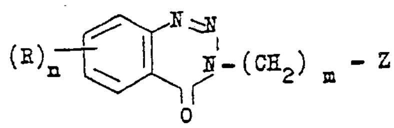

(IV)

est condensée sur une phényl-1 pipérazine convenablement substituée, de formule générale V

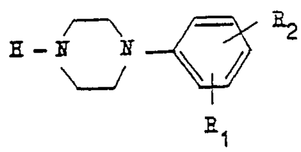

(V)

Dans les formules IV et V, R, $R_1$, $R_2$, m et n ont les significations données précédemment. Z représente un groupe facilement déplaçable par une amine et est de préférence un atome d'halogène ou un groupe tosyle (Ts) ou mésyle (Ms). La réaction s'effectue dans un solvant inerte comme l'acétonitrile, le N, N-diméthylformamide ou un alcanol de bas poids moléculaire. On peut opérer indifféremment avec un excès de phénylpipérazine V ou en présence d'un hydroxyde ou d'un carbonate alcalin. La température peut varier entre la température ambiante et la température d'ébullition du solvant utilisé. Le temps de réaction est généralement compris entre 10 et 60 heures.

Les dérivés IV s'obtiennent à partir des dérivés hydroxylés correspondants, de formule générale VI

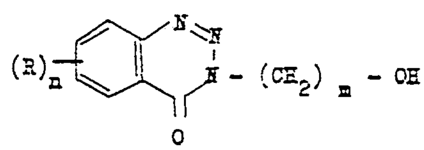

(VI)

dans laquelle R, m et n ont les significations données précédemment, selon des techniques classiques. Ainsi les dérivés IV pour lesquels Z = Cl s'obtiennent en traitant les alcools VI par le chlorure de thionyle. Les dérivés IV pour lesquels Z = Ts ou Z = Ms s'obtiennent en traitant les alcools VI par le chlorure de paratoluènesulfonyle ou par le chlorure de méthanesulfonyle, soit dans la pyridine soit dans un solvant tel que le chlorure de méthylène ou le chloroforme en présence d'une base comme la pyridine ou la triéthylamine.

Les dérivés VI pour lesquels m = 3 sont des composés intermédiaires nouveaux faisant partie de l'invention. Ils peuvent se préparer selon les méthodes suivantes :

α) traitement du sel de diazonium d'un dérivé de formule VII

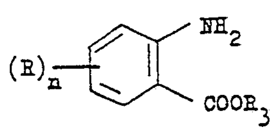

(VII)

dans laquelle R et n ont les mêmes significations que précédemment et $R_3$ est un radical alcoyle inférieur, par l'amino-3 propanol.

β) traitement d'un anhydride isatoïque de formule générale VIII

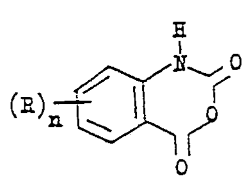

(VIII)

par l'amino-3 propanol pour conduire à un dérivé de formule générale IX

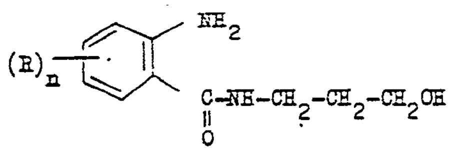

(IX)

lequel est cyclisé, sans isolement intermédiaire, par diazotation par un nitrite alcalin. Dans les formules VIII et IX, R et n ont les significations données précédemment.

On a constaté que les composés représentés par la formule I possèdent de remarquables propriétés sur le système nerveux central, qui les rendent utiles en médecine humaine dans le traitement des états dépressifs et des troubles psychiques.

Cette activité modificatrice de l'humeur peut être déterminée par des tests normalisés comme la potentialisation des effets centraux de l'hydroxy-5 tryptophane (5-HTP). Cette potentialisation objectivant le blocage axonal du recaptage de la sérotonine au niveau des terminaisons nerveuses sérotoninergiques est étudiée par la méthode décrite par T.M. Pugsley et X. Lippmann [Experientia *33*, 57 (1977)] : des souris Swiss sont traitées I.P. (par voie intra-péritonéale) avec les composés de l'étude, 30 minutes avant de recevoir 300 mg/kg de 5-HTP par voie I.P. Les souris sont observées 30 mn plus tard, pendant 1 mn, durant laquelle sont cotés les mouvements stéréotypés suivants : extension des pattes postérieures, tremblements, excitations, hochements de tête.

Les doses efficaces 50 (DE50) obtenues pour quelques produits de l'invention et celle obtenue pour le chlorhydrate de la TRAZODONE (chlorhydrate de [[(chloro-3 phényl)-4 pipérazinyl-1]-3 propyl]-2-2H-triazolo-1,2,4 [4,3-a]pyridinone-3) pris comme étalon sont consignées dans le tableau I :

Tableau I

| Produit | Potentialisation du 5-HTP DE50 (mg/kg/I.P) |
|---|---|
| TRAZODONE Chlorhydrate | 20 |
| Exemple 1 | 3 |
| Exemple 6 | 12 |
| Exemple 9 | 5 |
| Exemple 10 | 14 |
| Exemple 11 | 8 |
| Exemple 15 | 9 |
| Exemple 16 | 11 |
| Exemple 19 | 10 |
| Exemple 20 | 8 |
| Exemple 23 | 3 |
| Exemple 27 | 8 |
| Exemple 28 | 5 |
| Exemple 29 | 12 |

Les produits de l'invention manifestent une faible toxicité. Les doses léthales 50 (DL50) déterminées sur la souris Swiss par voie orale pour quelques uns des dérivés les plus actifs sont données dans le tableau II.

Tableau II

| Produit | D L 50 P.O (mg/kg) |
|---|---|
| TRAZODONE Chlorhydrate | 650 |
| Exemple 1 | > 3200 |
| Exemple 10 | 1500 |

Fortsetzung

| Produit | D L 50 P.O (mg/kg) |
|---|---|
| Exemple 11 | 1000 |
| Exemple 20 | > 3200 |
| Exemple 23 | 1060 |

La présente demande a également pour objet l'application des composés I à titre de médicaments et notamment de médicaments antidépresseurs. Ces médicaments peuvent être administrés par voie orale sous forme de comprimés, comprimés dragéifiés ou de gélules ou par voie rectale sous forme de suppositoires. Le principe actif est associé à divers excipients pharmaceutiquement compatibles. Les posologies journalières peuvent varier de 0,010 g à 0,5 g de principe actif selon l'âge du patient et la gravité de l'affection traitée.

On donne ci-dessous, à titre d'exemples non limitatifs quelques formulations pharmaceutiques :

Composition d'un comprimé de 100 mg éventuellement enrobé :

| | |
|---|---|
| principe actif | 10 mg |
| lactose | 40 mg |
| amidon de blé | 37 mg |
| gélatine | 2 mg |
| acide alginique | 5 mg |
| talc | 5 mg |
| stéarate de magnésium | 1 mg |

Composition d'une gélule :

| | |
|---|---|
| principe actif | 10 mg |
| lactose | 32 mg |
| amidon de blé | 25 mg |
| talc | 2,5 mg |
| stéarate de magnésium | 0,5 mg |

Composition d'un suppositoire de 3 g :

| | |
|---|---|
| principe actif | 20 mg |
| triglycérides semi-synthétiques q.s.p. | 3 g |

Les exemples suivants illustrent l'invention à titre non limitatif. Dans les données de résonance magnétique nucléaire (R.M.N.) les abréviations suivantes ont été utilisées : s pour singulet, t pour triplet et quint pour quintuplet.

## Exemple 1

[[(chloro-3 phényl)-4 pipérazinyl-1]-3 propyl]-3-3H-benzotriazine-1,2,3 one-4, chlorhydrate.

Un mélange de 90,5 g (0,615 mole) de 3H-benzotriazine-1,2,3 one-4 [préparée selon A. Weddige et H. Finger J. Prakt. Chem. *35*, 262, (1887)] de 168 g (0,615 mole) de (chloro-3 phényl)-1 (chloro-3 propyl)-4 pipérazine [préparée selon J. Bourdais Bull. Soc. Chim. France 3246 (1968)] de 86 g (0,615 mole) de carbonate de potassium et de 3 200 $cm^3$ d'acétonitrile est porté à reflux pendant 6 heures. Après refroidissement, les produits minéraux sont éliminés par filtration et le filtrat est concentré à sec sous pression réduite. Le résidu est filtré sur silice (éluant hexane-acétate d'éthyle 1/4). La concentration de l'éluat fournit la [[(chloro-3 phényl)-4 pipérazinyl-1]-3 propyl]-3-3H-benzotriazine-1,2,3 one-4. Elle est purifiée par recristallisation dans l'éthanol. Rdt : 156,1 g (66 %), F = 92-94 °C.

Analyse centésimale $C_{20}H_{22}ClN_5O$ (M = 383,88)

Calculé : C 62,57 H 5,78 Cl 9,24 N 18,24 %
Trouvé : C 62,70 H 5,63 Cl 9,49 N 18,00 %.

I. R. : $\bar{\nu}$ (C = O) = 1 685 $cm^{-1}$

R. M. N. ($CDCl_3$) $\delta$ = 2,1 (2H, quint) ; 2,4-2,8 (6H, massif complexe) ; 2,9-3,3 (4H, massif complexe) ; 4,6 (2H, t) 6,4-7,3 (4H, massif complexe) ; 7,6-8,6 (4H, massif complexe).

Le chlorhydrate de [[(chloro-3 phényl)-4 pipérazinyl-1]-3 propyl]-3-3H-benzotriazine-1,2,3 one-4 est

préparé par dissolution de la base dans l'éthanol, addition d'acide chlorhydrique 10 N puis évaporation à sec. Il est purifié par recristallisation dans l'éthanol. F = 201-203 °C

Analyse centésimale $C_{20}H_{23}Cl_2N_5O$ (M = 420,33)

Calculé : C 57,15 H 5,51 Cl 16,66 N 16,87 %
Trouvé : C 57,23 H 5,46 Cl 16,52 N 16,72 %.
I. R. $\bar{\nu}$ (C = O) = 1 670 $cm^{-1}$
R. M. N. (DMSO $d_6$) δ = 2,2-2,8 (2H, massif complexe) ; 2,8-4,2 (10H, massif complexe) ; 4,5 (2H, t) ; 6,7-8,4 (4H, massif complexe) ; 7,6-8,4 (4H, massif complexe) ; 12,7 (1H, pic échangeable avec $D_2O$).

## Exemple 2

[[(Chloro-3 phényl)-4 pipérazinyl-1]-3 propyl]-3-3H-benzotriazine-1,2,3 one-4.

Une solution de 170,2 g (0,623 mole) de (chloro-3 phényl)-1 (chloro-3 propyl)-4 pipérazine dans 230 $cm^3$ de N,N-diméthylformamide est ajoutée goutte à goutte, à 80 °C, à un mélange de 91,7 g (0,623 mole) de 3H-benzotriazine-1,2,3 one-4 de 87 g (0,623 mole) de carbonate de potassium et de 1 400 $cm^3$ de N,N-diméthylformamide. Le chauffage est poursuivi 1 heure après la fin de l'addition. Les produits minéraux sont alors filtrés et le filtrat concentré à sec sous pression réduite. Le résidu est filtré sur silice. L'évaporation de l'éluat fournit la [[(chloro-3 phényl)-4 pipérazinyl-1]-3 propyl]-3-3H-benzotriazine-1,2,3 one-4 identique à celle obtenue dans l'exemple 1. Rdt : 138,5 g (58 %) F = 92-94 °C (éthanol).

## Exemple 3

[[(chloro-3 phényl)-4 pipérazinyl-1]-3 propyl]-3-3H-benzotriazine-1,2,3 one-4.

a) (Hydroxy-3 propyl)-3-3H-benzotriazine-1,2,3 one-4

On ajoute par portions 53,8 g (0,33 mole) d'anhydride isatoïque à une solution de 32,3 g (0,43 mole) d'amino-3 propanol dans 120 $cm^3$ d'eau. La température monte spontanément jusqu'à 35 °C. Le mélange est porté 15 mn à reflux. Après refroidissement, on acidifie le milieu réactionnel par 130 $cm^3$ d'acide chlorhydrique à 33 % puis on ajoute goutte à goutte, entre 0 et 2 °C, une solution de 27,6 g (0,4 mole) de nitrite de sodium dans 75 $cm^3$ d'eau. Le milieu réactionnel est agité 30 mn à cette température, basifié par de l'ammoniaque et extrait plusieurs fois au chlorure de méthylène avec relargage de la phase aqueuse au chlorure de sodium. Les extraits organiques sont concentrés sous vide. Le résidu huileux obtenu est concrétisé par trituration dans un mélange d'hexane et de benzène. L'(hydroxy-3 propyl)-3-3H-benzotriazine-1,2,3 one-4 est purifiée par recristallisation dans un mélange hexane-acétate d'éthyle. Rdt : 75 %, F = 74-76 °C.

Analyse centésimale $C_{10}H_{11}N_3O_2$ (M = 205,21)

Calculé : C 58,53 H 5,40 N 20,48 %
Trouvé : C 58,71 H 5,48 N 20,09 %.
I. R. $\bar{\nu}$ (C = O) = 1 660 $cm^{-1}$ ; $\bar{\nu}$ (OH) = 3 400 $cm^{-1}$
R. M. N.($CDCl_3$) δ = 2,2 (2H, quint) ; 3,2 (1H, pic échangeable avec $D_2O$) ; 3,7 (2H,t) ; 4,7 (2H,t) ; 7,5-8,6 (4H, massif complexe)

Une autre méthode de synthèse est également possible :

Une solution de 27,4 g (0,4 mole) de nitrite de sodium dans 200 $cm^3$d'eau est ajoutée goutte à goutte entre 0 et 2 °C, à un mélange de 50 g (0,33 mole) d'anthranilate de méthyle, de 92,6 $cm^3$ (1 mole) d'acide chlorhydrique 33 % et de 300 $cm^3$ d'eau. Après 1 heure d'agitation, toujours entre 0 et 2 °C, on ajoute goutte à goutte 49,6 g (0,66 mole) d'amino-3 propanol. L'agitation est poursuivie encore 1 heure. Le milieu réactionnel est laissé revenir à température ambiante puis basifié par de l'ammoniaque. Le traitement se continue comme décrit ci-dessus. Rdt : 62 %.

b) (Chloro-3 propyl)-3-3H-benzotriazine-1,2,3 one-4

On ajoute 4,1 $cm^3$ (56 m. moles) de chlorure de thionyle à une solution de 9,5 g (46 m. moles) d'(hydroxy-3 propyl)-3-3H-benzotriazine-1,2,3 one-4 dans 110 $cm^3$ de chloroforme. Après 4 jours à température ambiante l'insoluble est filtré. Le filtrat est concentré sous pression réduite et le résidu concrétisé dans l'hexane. Il est purifié par recristallisation dans l'éthanol. Rdt : 51 %, F = 70-72 °C

Analyse centésimale $C_{10}H_{10}ClN_3O$ (M = 223,66)

Calculé : C 53,70 H 4,51 Cl 15,85 N 18,79 %
Trouvé : C 53,76 H 4,50 Cl 15,35 N 18,59 %.
I. R. $\bar{\nu}$ (C = O) = 1 680 $cm^{-1}$

c) [[(Chloro-3 phényl)-4 pipérazinyl-1]-3 propyl]-3-3H-benzotriazine-1,2,3 one-4, chlorhydrate

Une solution de 7,8 g (35 m. moles) de (chloro-3 propyl)-3-3H-benzotriazine-1,2,3 one-4, de 13 g (66 m. moles) de (chloro-3 phényl)-1 pipérazine et de 100 mg d'iodure de potassium dans 70 $cm^3$ d'acétonitrile est portée pendant 34 heures à reflux. Le chlorhydrate de (chloro-3 phényl)-1 pipérazine est filtré. Le filtrat est concentré sous pression réduite et le résidu purifié par filtration sur silice (éluant hexane-acétate d'éthyle 1/3). L'éluat est concentré sous vide et le résidu dissous dans l'éthanol. A cette solution on ajoute 6,9 $cm^3$ d'acide chlorhydrique à 33 %. Après concentration à sec, sous pression réduite, le chlorhydrate de [[(chloro-3 phényl)-4 pipérazinyl-1]-3 propyl]-3-3H-benzotriazine-1,2,3 one-4 est recristallisé dans l'éthanol. Rdt : 6,2 g (42 %), F = 201-203 °C. Le produit est identique à celui obtenu dans l'exemple 1.

Exemple 4

[[(Chloro-3 phényl)-4 pipérazinyl-1]-3 propyl]-3-3H-benzotriazine-1,2,3 one-4.

a) [(Méthyl-4 benzènesulfonyl)-3 propyl]-3-3H-benzotriazine-1,2,3 one-4

On ajoute par portions 20 g (105 m. moles) de chlorure de méthyl-4 benzènesulfonyle à une solution refroidie à 0 °C de 10,7 g (52 m. moles) d'(hydroxy-3 propyl)3-3H-benzotriazine-1,2,3 one-4 (préparée comme il est décrit dans l'exemple 3) dans 250 $cm^3$ de pyridine. Le milieu réactionnel est agité 4 heures à 0 °C puis abandonné 24 heures à cette température. Il est ensuite versé dans 2 l d'eau glacée. Le précipité formé est filtré, lavé à l'eau et séché. Il est recristallisé dans un mélange d'hexane et d'acétate d'éthyle. Rdt : 12,6 g (67 %), F = 76-77 °C

Analyse centésimale $C_{17}H_{17}N_3O_4S$ (M = 359,40)

Calculé : C 56,81 H 4,77 N 11,69 S 8,92 %
Trouvé : C 56,76 H 4,80 N 11,67 S 8,88 %.
I. R. $\bar{\nu}$ (C = O) = 1 680 $cm^{-1}$
R. M. N. (DMSO $d_6$) $\delta$ = 2,2 (2H, quint) ; 2,4 (3H,s) ; 4,2 (2H,t) ; 4,5 (2H,t) ; 7,4-7,9 (4H,2d) ; 7,9-8,5 (4H, massif complexe).

b) [[(Chloro-3 phényl)-4 pipérazinyl-1]-3 propyl]-3-3H-benzotriazine-1,2,3 one-4

Un mélange de 7,2 g (20 m. moles) de [(méthyl-4 benzènesulfonyl)-3 propyl]-3-3H-benzotriazine-1,2,3 one-4, de 3,9 g (20 m. moles) de (chloro-3 phényl)-1 pipérazine, de 3,1 g (22 m. moles) de carbonate de potassium et de 50 $cm^3$ de N,N-diméthylformamide est agité 8 heures à température ambiante puis 1 heure à 80 °C. Après refroidissement, le milieu réactionnel est versé dans 500 $cm^3$ d'eau. Le précipité est filtré et recristallisé dans l'éthanol. On obtient 4,0 g (rdt : 52 %) de [[(chloro-3 phényl)-4 pipérazinyl-1]-3 propyl]-3-3H-benzotriazine-1,2,3 one-4 identique à celle obtenue dans les exemples précédents.

Exemple 5

[[(chloro-3 phényl)-4 pipérazinyl-1]-3 propyl]-3-3H benzotriazine-1,2,3 one-4.

a) (Méthanesulfonyl-3 propyl)-3-3H-benzotriazine-1,2,3 one-4

On ajoute à 0 °C, 16,2 g (0,16 mole) de triéthylamine à une solution de 22,1 g (0,107 mole) d'(hydroxy-3 propyl)-3-3H-benzotriazine-1,2,3 one-4 dans 535 $cm^3$ de chlorure de méthylène puis goutte à goutte, toujours à 0 °C, 15,9 g (0,139 mole) de chlorure de méthanesulfonyle. L'agitation est poursuivie 20 mn puis le milieu réactionnel est versé sur de la glace. La phase organique est décantée, lavée par une solution saturée de bicarbonate de sodium et séchée sur sulfate de sodium. Après évaporation du solvant, le résidu est concrétisé dans l'hexane et recristallisé dans un mélange d'hexane et d'acétate d'éthyle. Rdt : 23,6 g (78 %), F = 102-103 °C.

Analyse centésimale $C_{11}H_{13}N_3O_4S$ (M = 283,30)

Calculé : C 46,63 H 4,62 N 14,83 S 11,32 %
Trouvé : C 46,66 H 4,60 N 14,74 S 11,35 %.
I. R. $\bar{\nu}$ (C = O) = 1 660 $cm^{-1}$
R. M. N. ($CDCl_3$) $\delta$ = 2,2 (2H, quint) ; 2,9 (3H,s) ; 4,2 (2H,t) ; 4,5 (2H,t) ; 7,4-8,3 (4H, massif complexe)

b) [[(Chloro-3 phényl)-4 pipérazinyl-1]-3 propyl]-3-3H-benzotriazine-1,2,3 one-4

Un mélange de 11,3 g (40 m. moles) de (méthanesulfonyl-3 propyl)-3-3H-benzotriazine-1,2,3 one-4, de 7,8 g (40 m. moles) de (chloro-3 phényl)-1 pipérazine, de 6,2 g (44 m. moles) de carbonate de potassium et de 100 $cm^3$ de N,N-diméthylformamide est agité à température ambiante pendant 2 heures puis à 90 °C pendant 6 heures. Après refroidissement, le milieu réactionnel est repris par de l'eau et extrait à l'éther. La phase organique est concentrée et le résidu recristallisé dans l'éthanol. On obtient 3,7 g (Rdt : 24 %) de [[(chloro-3 phényl)-4 pipérazinyl-1]-3 propyl]-3-3H-benzotriazine-1,2,3 one-4 identique à celle obtenue dans les exemples précédents.

Exemple 6

[[(Chloro-2 phényl-4 pipérazinyl-1]-3 propyl]-3-3H-benzotriazine-1,2,3 one-4, chlorhydrate.

En opérant comme dans l'exemple 1, à partir de 4,4 g (30 m. moles) de 3H-benzotriazine-1,2,3 one-4, de 8,2 g (30 m. moles) de (chloro-2 phényl)-1 (chloro-3 propyl)-4 pipérazine (préparée selon C. B. Pollard et coll. J ; Org. Chem. *24*, 764 (1959)], de 8,4 g (60 m. moles) de carbonate de potassium et de 150 $cm^3$ d'acétonitrile, on obtient 6,1 g (rdt 48 %) de chlorhydrate de [[(chloro-2 phényl)-4 pipérazinyl-1]-3 propyl]-3-3H-benzotriazine-1,2,3 one-4, F = 232-234 °C (éthanol).

Analyse centésimale $C_{20}H_{23}Cl_2N_5O$ (M = 420,33)

Calculé : C 57,15 H 5,51 Cl 16,87 N 16,66 %
Trouvé : C 56,97 H 5,40 Cl 16,88 N 16,49 %.
I. R. $\bar{\nu}$ (C = O) = 1 690 $cm^{-1}$

Exemple 7

[[(Trifluorométhyl-3 phényl)-4 pipérazinyl-1]-3 propyl]-3-3H-benzotriazine-1,2,3 one-4, chlorhydrate.

Obtenu en opérant comme dans l'exemple 3 à partir de 6,2 g (28 m. moles) de (chloro-3 propyl)-3-3H-benzotriazine-1,2,3 one-4, de 12 g (46 m. moles) de chlorhydrate de (trifluorométhyl-3 phényl)-1 pipérazine [préparée selon A.F. Ash et coll. brevet britannique 948.765 ; C.A. *60*, 12029a (1964)], de 23,4 g (170 m. moles) de carbonate de potassium, de 100 mg d'iodure de potassium et de 80 $cm^3$ d'acétonitrile. Rdt : 5,0 g (39 %), F = 208-210 °C (éthanol).

Analyse centésimale $C_{21}H_{23}ClF_3N_5O$ (M = 453,89)

Calculé : C 55,57 H 5,11 Cl 7,81 F 12,56 N 15,43 %
Trouvé : C 55,68 H 5,06 Cl 7,48 F 12,65 N 15,33 %.
I. R. $\bar{\nu}$ (C = O) = 1 680 $cm^{-1}$

Exemple 8

[[(Chloro-4 phényl)-4 pipérazinyl-1]-3 propyl]-3-3H-benzotriazine-1,2,3 one-4, chlorhydrate.

Obtenu en opérant comme dans l'exemple 3 à partir de 5,6 g (25 m. moles) de (chloro-3 propyl)-3-3H-benzotriazine-1,2,3 one-4, de 9,8 g (50 m. moles) de (chloro-4 phényl)-1 pipérazine, de 7,1 g (51 m. moles) de carbonate de potassium, de 100 mg d'iodure de potassium et de 80 $cm^3$ d'acétonitrile. Rdt : 6,0 g (57 %), F = 232-234 °C (éthanol).

Analyse centésimale $C_{20}H_{23}Cl_2N_5O$ (M = 420,33)

Calculé : C 57,15 H 5,51 Cl 16,87 N 16,66 %
Trouvé : C 56,95 H 5,60 Cl 16,97 N 16,80 %.
I. R. $\bar{\nu}$ (C = O) = 1 680 $cm^{-1}$

Exemple 9

[[(Méthyl-2 phényl)-4 pipérazinyl-1]-3 propyl]-3-3H-benzotriazine-1,2,3 one-4, chlorhydrate.

Obtenu en opérant comme dans l'exemple 1 à partir de 6,4 g (43,5 m. moles) de 3H-benzotriazine-1,2,3 one-4, de 11 g (43,5 m. moles) de (chloro-3 propyl)-1 (méthyl-2 phényl)-4 pipérazine [ préparée selon C. B. Pollard et coll. J. Org. Chem. *24*, 764 (1959)], de 12,2 g (87 m. moles) de carbonate de potassium et de 220 $cm^3$ d'acétonitrile. Rdt : 9,4 g (54 %). F = 215-217 °C (éthanol).

Analyse centésimale $C_{21}H_{26}ClN_5O$ (M = 399,92)

Calculé : C 63,07 H 6,55 Cl 8,87 N 17,51 %
Trouvé : C 63,21 H 6,64 Cl 9,18 N 17,42 %.
I. R. $\bar{\nu}$ (C = O) = 1 685 $cm^{-1}$

Exemple 10

[[(Fluoro-3 phényl)-4 pipérazinyl-1]-3 propyl]-3H-benzotriazine-1,2,3 one-4, chlorhydrate.

Obtenu en opérant comme dans l'exemple 1 à partir de 4,6 g (31 m. moles) de 3H-benzotriazine-1,2,3 one-4, de 8 g (31 m. moles) de (chloro-3 propyl)-1 (fluoro-3 phényl)-4 pipérazine, de 8,7 g (63 m. moles) de carbonate de potassium et de 155 $cm^3$ d'acétonitrile. Rdt : 7,1 g (56 %) F = 225-226 °C.

Analyse centésimale $C_{20}H_{23}ClFN_5O$ (M = 403,88)

Calculé : C 59,47 H 5,74 Cl 8,78 F 4,70 N 17,34 %
Trouvé : C 59,75 H 5,75 Cl 8,51 F 4,73 N 17,35 %.
I. R. $\bar{\nu}$ (C = O) = 1 665 $cm^{-1}$

La (chloro-3 propyl)-1 (fluoro-3 phényl)-4 pipérazine utilisée est préparée de la manière suivante : 32 g (203 m. moles) de bromo-1 chloro-3 propane sont ajoutés goutte à goutte à un mélange de 12,2 g (67,7 m. moles) de (fluoro-3 phényl)-1 pipérazine [préparée selon D. R. Maxwell et W. R. Wragg, brevet anglais 943.739 ; C.A. *60*, 5522c (1964)] de 9,4 g (68 m. moles) de carbonate de potassium et de 135 $cm^3$ de N,N-diméthylformamide. L'agitation est poursuivie 9 heures à température ambiante. Les produits minéraux sont alors éliminés par filtration puis le filtrat est concentré sous pression réduite. Le résidu est dissous dans l'éther, la solution lavée avec une solution saturée de chlorure de sodium puis séchée sur sulfate de sodium. L'évaporation de l'éther fournit une huile qui est distillée ($Eb_1$ = 137-140 °C). On obtient la (chloro-3 propyl)-1 (fluoro-3 phényl)-4 pipérazine sous forme d'une huile que l'on cristallise dans l'hexane. Rdt : 11,6 g (67 %), F = 48-49 °C.

Analyse centésimale $C_{13}H_{18}ClN_2$ (M = 256,75)

Calculé : C 60,81 H 7,07 Cl 13,81 F 7,40 N 10,91 %
Trouvé : C 61,15 H 7,14 Cl 13,90 F 7,45 N 10,94 %.

Exemple 11

[(Phényl-4 pipérazinyl-1)-3 propyl)-3-3H-benzotriazine-1,2,3 one-4, chlorhydrate.

Obtenu en opérant comme dans l'exemple 1 à partir de 8,8 g (60 m. moles) de 3H-benzotriazine-1,2,3 one-4 de 14,3 g (60 m. moles) de (chloro-3 propyl)-1 phényl-4 pipérazine, de 16,6 g (120 m. moles) de carbonate de potassium et de 300 $cm^3$ d'acétonitrile. Rdt : 12,7 g (55 %) F = 205-207 °C (éthanol).

Analyse centésimale $C_{20}H_{24}ClN_50$ (M = 385,89)

Calculé : C 62,25 H 6,27 Cl 9,19 N 18,15 %
Trouvé : C 62,18 H 6,42 Cl 9,12 N 18,22 %.
I. R. $\bar{\nu}$ (C = O) = 1 680 $cm^{-1}$

Exemple 12

[[(Méthoxy-2 phényl)-4 pipérazinyl-1]-3 propyl]-3-3H-benzotriazine-1,2,3 one-4.

Obtenu en opérant comme dans l'exemple 1 à partir de 4,2 g (29 m. moles) de 3H-benzotriazine-1,2,3 one-4, de 7,7 g (29 m. moles) de (chloro-3 propyl)-1 (méthoxy-2 phényl)-4 pipérazine [préparée selon J. Bourdais Bull. Soc. Chim. France 3246, (1968)], de 8,0 g (58 m. moles) de carbonate de potassium et de 145 $cm^3$ d'acétonitrile. Rdt : 5,9 g (54 %), F = 86-88 °C (hexane-acétate d'éthyle).

Analyse centésimale $C_{21}H_{25}N_5O_2$ (379,45)

Calculé : C 66,47 H 6,64 N 18,46 %
Trouvé : C 66,56 H 6,65 N 18,28 %.
I. R. $\bar{\nu}$ (C = O) = 1 690 cm$-{}^{1}$

Exemple 13

[[(Méthyl-3 phényl)-4 pipérazinyl-1]-3 propyl]-3-3H-benzotriazine-1,2,3 one-4.

Obtenu en opérant comme dans l'exemple 2 à partir de 5,9 g (40 m. moles) de 3H-benzotriazine-1,2,3 one-4, de 10,1 g (40 moles) de (chloro-3 propyl)-1 (méthyl-3 phényl)-4 pipérazine, de 5,6 g (40 m. moles) de carbonate de potassium et de 100 $cm^3$ de N,N-diméthylformamide. Rdt : 9,0 g (62 %), F = 72-73 °C (pentane-acétate d'éthyle).

Analyse centésimale $C_{21}H_{25}N_5O$ (M = 363,45)

Calculé : C 69,39 H 6,93 N 19,27 %
Trouvé : C 69,38 H 6,93 N 19,40 %.
I. R. $\bar{\nu}$ (C = O) = 1 690 $cm^{-1}$

La (chloro-3 propyl)-1 (méthyl-3 phényl)-4 pipérazine utilisée est préparée de la manière suivante : 21,3 g (136 m. moles) de bromo-1 chloro-3 propane sont ajoutés rapidement, sous agitation, à un mélange de 20 g (113 m. moles) de (méthyl-3 phényl)-1 pipérazine [préparée selon C. B. Pollard et T. Wicker Jr. J. Am. Chem. Soc. *76*, 1853 (1954)], de 18,7 g (136 m. moles) de carbonate de potassium et de 110 $cm^3$ de N,N-diméthylformamide. L'agitation est poursuivie pendant 5 heures à température ambiante. Les produits minéraux sont alors filtrés et le filtrat concentré sous pression réduite. Le résidu est dissous dans de l'éther, la solution est lavée avec une solution saturée de chlorure de sodium puis séchée sur sulfate de sodium. Après évaporation de l'éther, le résidu est distillé. $Eb_{2\text{-}3}$ = 156-160 °C, Rdt : 14,7 g 52 %).

R.M.N. ($CDCl_3$) δ = 2,0 (2H, quint) ; 2,3 (3H, s) ; 2,4-2,8 (6H, massif complexe) ; 3,1-3,4 (4H, massif complexe) 3,6 (2H, t) ; 6,6-6,9 (3H, massif complexe) ; 7,0-7,4 (1H, massif complexe).

## Exemple 14

[[(Méthyl-4 phényl)4 pipérazinyl-1]-3 propyl]-3-3H-benzotriazine-1, 2,3 one-4.

Obtenu en opérant comme dans l'exemple 1, à partir de 4,4 g (30 m. moles) de 3H-benzotriazine-1,2,3 one-4, de 7,6 g (30 m. moles) de (chloro-3 propyl)-1 (méthyl-4 phényl)-4 pipérazine, de 8,4 g (60 m. moles) de carbonate de potassium et de 150 $cm^3$ d'acétonitrile. Rdt : 6,8 g (62 %), F = 86-87 °C (hexane-acétate d'éthyle).

Analyse centésimale $C_{21}H_{25}N_5O$ (M = 363,45)

Calculé : C 69,39 H 6,93 N 19,27 %
Trouvé : C 69,24 H 6,94 N 19,22 %.
I. R. $\bar{\nu}$ (C = O) = 1 690 $cm^{-1}$

La (chloro-3 propyl)-1 (méthyl-4 phényl)-4 pipérazine utilisée est obtenue en opérant comme dans l'exemple 13, à partir de 13,2 g (84 m. moles) de bromo-1 chloro-3 propane, de 12,3 g (70 m. moles) de (méthyl-4 phényl)-1 pipérazine [préparée selon C. B. Pollard et T. H. Wicker Jr. J. Am. Chem. Soc. *76*, 1853 (1954)], de 11,6 g (84 m. moles) de carbonate de potassium et de 70 $cm^3$ de N,N-diméthylformamide. Rdt : 7,6 g (43 %), $Eb_{1\text{-}3}$ = 150-160 °C.

## Exemple 15

[[Méthoxy-3 phényl)-4 pipérazinyl-1]-3 propyl]-3-3H-benzotriazine-1, 2,3 one-4.

Obtenu en opérant comme dans l'exemple 1 à partir de 5,1 g (35 m. moles) de 3H-benzotriazine-1,2,3 one-4, de 9,4 g (35 m. moles) de (chloro-3 propyl)-1 (méthoxy-3 phényl)-4 pipérazine, de 9,7 g (70 m. moles) de carbonate de potassium et de 200 $cm^3$ d'acétonitrile. Rdt : 8,2 g (62 %), F = 63-65 °C (pentane-acétate d'éthyle).

Analyse centésimale $C_{21}H_{25}N_5O_2$ (M = 379,45)

Calculé : C 66,47 H 6,64 N 18,46 %
Trouvé : C 66,18 H 6,56 N 18,34 %.
I. R. $\bar{\nu}$ (C = O) = 1 690 $cm^{-1}$

La (chloro-3 propyl)-1 (méthoxy-3 phényl)-4 pipérazine utilisée est préparée en opérant comme dans l'exemple 13, à partir de 11,8 g (75 m. moles) de bromo-1 chloro-3 propane, de 12,0 g (62,5 m. moles) de (méthoxy-3 phényl)-1 pipérazine [préparée selon P. C. Jain et coll. J. Med. Chem. *10*, 812 (1967)], de 10,4 g (75 m. moles) de carbonate de potassium et de 65 $cm^3$ de N,N-diméthylformamide. Rdt : 9,4 g (56 %), $Eb_{2\text{-}3}$ = 175-190 °C.

Exemple 16

[[(Méthoxy-4 phényl)-4 pipérazinyl-1]-3 propyl]-3-3H-benzotriazine-1, 2,3 one-4

Obtenu en opérant comme dans l'exemple 1 à partir de 4,1 g (28 m. moles) de 3H-benzotriazine-1,2,3 one-4, de 7,5 g (28 m. moles) de (chloro-3 propyl)-1 (méthoxy-4 phényl)-4 pipérazine [préparée selon J. Bourdais, Bull. Soc. Chim. France 3246, (1968)], de 7,8 g (56 m. moles) de carbonate de potassium et de 140 $cm^3$ d'acétonitrile. Rdt : 4,5 g (42 %), F = 111-113 °C (hexane-acétate d'éthyle).

Analyse centésimale $C_{21}H_{25}N_5O_2$ (M = 379,45).

Calculé : C 66,47 H 6,64 N 18,46 %
Trouvé : C 66,71 H 6,70 N 18,30 %.
I. R. $\bar{\nu}$ (C = O) = 1 685 $cm^{-1}$

Exemple 17

Chloro-6[[(chloro-3 phényl)-4 pipérazinyl-1]-3 propyl]-3-3H-benzotriazine-1,2,3 one-4, chlorhydrate.

Obtenu selon la suite de réactions décrites dans l'exemple 3.

Etape a

Chloro-6 (hydroxy-3 propyl)-3-3H-benzotriazine-1,2,3 one-4

On ajoute par portions 48,9 g (0,25 mole) d'anhydride chloro-5 isatoïque à une solution de 24,8 g (0,33 mole) d'amino-3 propanol-1 dans 90 $cm^3$ d'eau. Le mélange est porté 15 mn à reflux. Après refroidissement on acidifie le milieu réactionnel par 100 $cm^3$ d'acide chlorhydrique 33 % puis on ajoute, entre 0 et 2 °C, une solution de 21 g (0,3 mole) de nitrite de sodium dans 60 $cm^3$ d'eau. Le milieu réactionnel est agité 30 mn à cette température puis basifié par de l'ammoniaque. Le précipité obtenu est filtré,séché et recristallisé dans un mélange d'hexane et d'acétate d'éthyle. Rdt : 44,5 g (74 %), F = 102-104,5 °C.

Analyse centésimale $C_{10}H_{10}ClN_3O_2$ (M = 239,66)

Calculé : C 50,11 H 4,21 Cl 14,79 N 17,53 %
Trouvé : C 49,96 H 4,12 Cl 14,52 N 17,40 %.
I. R. $\bar{\nu}$ (C = O) = 1 670 $cm^{-1}$. $\nu$ (OH) = 3 420 $cm^{-1}$.
R.M.N. ($CDCl_3$) δ = 2,2 (2H, quint) ; 2,8 (1H, s. échangeable à $D_2O$) ; 3,8 (2H,t) ; 4,7 (2H,t) ; 7,8-8,5 (3H, massif complexe).

Etape b

Chloro-6 (chloro-3 propyl)-3-3H-benzotriazine-1,2,3 one-4

A partir de 24 g (0,1 mole) de chloro-6 (hydroxy-3 propyl)-3-3H-benzotriazine-1,2,3 one-4 de 8,7 $cm^3$ (0,12 mole) de chlorure de thionyle et de 400 $cm^3$ de chloroforme, on obtient 10 g de produit. Rdt : 39 %, F = 102-105 °C (éthanol).

I. R. $\bar{\nu}$ (C = O) = 1 660 $cm^{-1}$. R. M. N. ($CDCl_3$) δ = 2,4 (2H, quint) ; 4,2 (2H, t) ; 4,7 (2H, t) ; 7,8-8,6 (3H, massif complexe).

Etape c

Chloro-6[[(chloro-3 phényl)-4 pipérazinyl-1]-3 propyl]-3-3H-benzotriazine-1,2,3 one-4, chlorhydrate.

Un mélange de 3,2 g (12 m. moles) de chloro-6 (chloro-3 propyl)-3-3H-benzotriazine-1,2,3 one-4, de 6,5 g (24 m. moles) de chlorhydrate de (chloro-3 phényl)-1 pipérazine, de 10,2 g (72 m. moles) de carbonate de potassium, de 50 mg d'iodure de potassium et de 50 $cm^3$ d'acétonitrile est porté 38 heures à reflux. Après refroidissement le milieu réactionnel est dilué avec 200 $cm^3$ d'eau et extrait au chlorure de méthylène. La solution organique est concentrée à sec et le résidu purifié par filtration sur silice (éluant hexane-acétate d'éthyle 1/3). La concentration de l'éluat sous pression réduite fournit un résidu qui est dissous dans l'éthanol. A cette solution on ajoute 1,5 $cm^3$ d'acide chlorhydrique 33 % et évapore à sec. Le chlorhydrate de chloro-6 [[(chloro-3 phényl)-4 pipérazinyl-1]-3 propyl]-3-3H-benzotriazine-1,2,3 one-4 est recristallisé dans l'éthanol. Rdt : 1,0 g (18 %), F = 228-230,5 °C.

Analyse centésimale $C_{20}H_{22}Cl_3N_5O$ (M = 454,79).

Calculé : C 52,82 H 4,88 Cl 23,39 N 15,40 %
Trouvé : C 52,65 H 4,76 Cl 23,47 N 15,34 %.
I. R. $\bar{\nu}$ (C = O) = 1 670 $cm^{-1}$

Exemple 18

Chloro-6[[(chloro-3 phényl)-4 pipérazinyl-1]-3 propyl]-3-3H-benzotriazine-1,2,3 one-4, chlorhydrate.

En opérant comme dans l'exemple 1, à partir de 3,6 g (20 m. moles) de chloro-6-3H-benzotriazine-1,2,3 one-4 [préparée selon S. M. Gadekar et E. Ross J. Org. Chem. *26,* 613 (1961)], de 5,5 (20 m. moles) de (chloro-3 phényl)-1 (chloro-3 propyl)-4 pipérazine, de 5,6 g (40 m. moles) de carbonate de potassium et de 100 $cm^3$ d'acétonitrile, on obtient 5,6 g (rdt : 62 %) du produit désiré, identique à celui obtenu dans l'exemple 17.

Exemple 19

Chloro-7[[(chloro-3 phényl)-4 pipérazinyl-1]-3 propyl]-3-3H-benzotriazine-1,2,3 one-4, chlorhydrate.

Obtenu en opérant comme dans l'exemple 1 à partir de 6,4 g (35 m. moles) de chloro-7-3H-benzotriazine-1,2,3 one-4 [préparée selon S. M. Gadekar et E. Ross J. Org. Chem. *26,* 613 (1961)], de 9,6 g (35 m. moles) de (chloro-3 phényl)-1 (chloro-3 propyl)-4 pipérazine, de 9,9 g (70 m. moles) de carbonate de potassium et de 175 $cm^3$ d'acétonitrile. Rdt : 8,7 g (55 %), F = 218-220 °C (éthanol).

Analyse centésimale $C_{20}H_{22}Cl_3N_5O$ (M = 454,79)

Calculé : C 52,82 H 4,88 Cl 23,39 N 15,40 %
Trouvé : C 52,86 H 5,05 Cl 23,33 N 15,49 %.
I. R. $\bar{\nu}$ (C = O) = 1 680 $cm^{-1}$

Exemple 20

[[(chloro-3 phényl)-4 pipérazinyl-1]-3 propyl]-3 méthyl-6-3H-benzotriazine-1,2,3 one-4, chlorhydrate.

Obtenu en opérant comme dans l'exemple 1 à partir de 7,6 g (47 m. moles) de méthyl-6-3H-benzotriazine-1,2,3 one-4, de 12,9 g (47 m. moles) de (chloro-3 phényl)-1 (chloro-3 propyl)-4 pipérazine, de 13,0 g (94 m. moles) de carbonate de potassium et de 245 $cm^3$ d'acétonitrile. Rdt : 10,8 g (53 %), F = 232-234 °C (éthanol-DMF).

Analyse centésimale $C_{21}H_{25}Cl_2N_5O$ (M = 434,36)

Calculé : C 58,06 H 5,80 Cl 16,33 N 16,13 %
Trouvé : C 57,95 H 5,78 Cl 16,41 N 16,12 %.
I. R. $\bar{\nu}$ (C = O) = 1 670 $cm^{-1}$.

La méthyl-6-3H-benzotriazine-1,2,3 one-4 utilisée a été préparée de la manière suivante : une solution de 5,5 g (79 m. moles) de nitrite de sodium dans 55 $cm^3$ d'eau est ajoutée goutte à goutte, entre 0 et 2 °C à un mélange de 10 g (66 m. moles) d'amino-2 méthyl-5 benzamide [préparé selon K. Wasti et coll. Synthesis 570 (1974)], de 49,5 $cm^3$ d'acide chlorhydrique 33 % et de 115 $cm^3$ d'eau. L'agitation est poursuivie à cette température, 1 heure après la fin de l'addition. Le milieu réactionnel est ensuite basifié par 35 $cm^3$ d'ammoniaque 34 %. Le précipité obtenu est filtré, lavé à l'eau, séché puis recristallisé dans de l'éthanol. Rdt : 7,6 g (60 %), F = 219-220 °C.

Analyse centésimale $C_8H_7N_3O$ (M = 161,16)

Calculé : C 59,62 H 4,38 N 26,07 %
Trouvé : C 59,35 H 4,42 N 26,06 %.
I. R. $\bar{\nu}$ (C = O) 1 665 $cm^{-1}$

Exemple 21

[[(Chloro-3 phényl)-4 pipérazinyl-1]-3 propyl]-3 méthoxy-6-3H-benzotriazine-1,2,3 one-4, chlorhydrate.

Obtenu en opérant comme dans l'exemple 1 à partir de 9 g (51 m. moles) de méthoxy-6-3H-benzotriazine-1,2,3 one-4 [préparée selon J. G. Archer et coll. J. Chem. Soc. Perkin Trans. 1 1169 (1973)], de 13,9 g (51 m. moles) de (chloro-3 phényl)-1 (chloro-3 propyl)-4 pipérazine, de 14,2 g (103 m. moles) de carbonate de potassium et de 155 $cm^3$ d'acétonitrile. Rdt : 8,3 g (36 %), F = 211-213 °C (éthanol).

Analyse centésimale $C_{21}H_{25}Cl_2N_5O$ (M = 450,36)

Calculé : C 56,00 H 5,60 Cl 15,75 N 15,55 %
Trouvé : C 55,73 H 5,77 Cl 15,95 15,26 %.
I. R. $\bar{\nu}$ (C = O) = 1 675 $cm^{-1}$

Exemple 22

[[(Chloro-3 phényl)-4 pipérazinyl-1]-3 propyl]-3 diméthoxy-6,7-3H-benzotriazine-1,2,3 one-4, chlorhydrate.

Obtenu en opérant comme dans l'exemple 1 à partir de 5,8 g (28 m. moles) de diméthoxy-6,7-3H-benzotriazine-1,2,3 one-4 [préparée selon F. G. Kathawala, brevet américain 3.678.166], de 7,7 g (28 m. moles) de (chloro-3 phényl)-1 (chloro-3 propyl)-4 pipérazine, de 3,9 g (28 m. moles) de carbonate de potassium et de 140 $cm^3$ d'acétonitrile. Rdt : 4,6 g (37 %), F = 235-237 °C (éthanol).

Analyse centésimale $C_{22}H_{27}Cl_2N_5O_3$ (M = 443,93)

Calculé : C 55,00 H 5,67 Cl 14,76 N 14,58 %
Trouvé : C 54,64 H 5,80 Cl 14,73 N 14,40 %.
I. R. $\bar{\nu}$ (C = O) = 1 675 $cm^{-1}$

Exemple 23

[[(Chloro-3 phényl)-4 pipérazinyl-1]-3 propyl]-3-3H-thiéno [2,3-d]triazine-1,2,3 one-4, chlorhydrate.

Obtenu en opérant comme dans l'exemple 1 à partir de 4,1 g (27 m. moles) de 3H-thiéno [2,3-d] triazine-1,2,3 one-4 [préparée selon F. Sauter et W. Deinhammer Monatsch. Chem. *104,* 1586 (1973)], de 7,4 g (27 m. moles) de (chloro-3 phényl)-1 (chloro-3 propyl)-4 pipérazine, de 4,2 g (30 m. moles) de carbonate de potassium et de 130 $cm^3$ d'acétonitrile. Rdt : 4,9 (43 %), F = 220-224 °C (éthanol).

Analyse centésimale $C_{18}H_{21}Cl_2N_5OS$ (M = 426,37)

Calculé : C 50,70 H 4,97 Cl 16,63 N 16,43 S 7,52 %
Trouvé : C 50,46 H 4,96 Cl 16,37 N 16,13 S 7,25 %.
I. R. $\bar{\nu}$ (C = O) = 1 695 $cm^{-1}$

Exemple 24

[[(Chloro-3 phényl)-4 pipérazinyl-1]-3 propyl]-3 tétrahydro-5,6,7,8-3H-benzo [1]-thiéno [2,3-d]triazine-1,2,3 one-4, chlorhydrate.

Obtenu en opérant comme dans l'exemple 1 à partir de 5,2 g (25 m. moles) de tétrahydro-5,6,7,8-3H-benzo [1] thiéno [2,3-d] triazine-1,2,3 one-4 [préparée selon F. Sauter et W. Deinhammer Monatsh. Chem. *104,* 1586 (1973)], de 6,8 g (25 m. moles) de (chloro-3 phényl)-1 (chloro-3 propyl)-4 pipérazine, de 7,0 g (50 m. moles) de carbonate de potassium et de 125 $cm^3$ d'acétonitrile. Rdt : 7,0 g (63 %), F = 163-164,5 °C (éthanol).

Analyse centésimale $C_{22}H_{27}Cl_2N_5OS$ (M = 480,46)

Calculé : C 54,99 H 5,67 Cl 14,76 N 14,58 S 6,67 %
Trouvé : C 54,81 H 5,70 Cl 14,58 N 14,64 S 6,79 %.
I. R. $\bar{\nu}$ (C = O) = 1 670 $cm^{-1}$

Exemple 25

[[(Chloro-3 phényl)-4 pipérazinyl-1]-3 propyl]-3 nitro-6-3H-benzotriazine-1,2,3 one-4, chlorhydrate.

Obtenu en opérant comme dans l'exemple 1, à partir de 10 g (52 m. moles) de nitro-6-3H-benzotriazine-1,2,3 one-4 [préparée selon J. Adamson et coll. J. Chem. Soc. C, 981 (1971)], de 14,8 g (54 m. moles) de (chloro-3 phényl)-1 (chloro-3 propyl)-4 pipérazine, de 7,2 g (52 m. moles) de carbonate de potassium et de 260 $cm^3$ d'acétonitrile. Rdt : 4,5 g (19 %), F = 239-241 °C (éthanol).

Analyse centésimale $C_{20}H_{22}Cl_2N_6O_3$ (M = 465,34)

Calculé : C 51,62 H 4,77 Cl 15,24 N 18,06 %
Trouvé : C 51,58 H 4,84 Cl 15,12 N 18,32 %.
I. R. $\bar{\nu}$ (C = O) = 1 690 $cm^{-1}$

Exemple 26

Chloro-8[[(cloro-3)phényl-4 pipérazinyl-1]-3 propyl]-3-3H benzotriazine-1,2,3 one-4, chlorhydrate.

Obtenu en opérant comme dans l'exemple 1, à partir de 7,8 g (43 m. moles) de chloro-8-3H-benzotriazine-1,2,3 one-4 [préparée selon la technique décrite dans : C. A. *68,* 114665e (1968)], de 11,8 g (43 m. moles) de (chloro-3 phényl)-1 (chloro-3 propyl)-4 pipérazine, de 12 g (87 m. moles) de carbonate de potassium et de 130 $cm^3$ d'acétonitrile. Rdt : 7,4 g (38 %), F = 225-227 °C (éthanol-DMF).

Analyse centésimale $C_{20}H_{22}Cl_3N_5O$ (M = 454,79)

Calculé : C 52,82 H 4,88 Cl 23,39 N 15,40 %
Trouvé : C 53,11 H 4,91 Cl 23,39 N 15,39 %.
I. R. $\bar{\nu}$ (C=O) = 1 675 $cm^{-1}$

Exemple 27

[(Chloro-3 fluoro-4 phényl)-4 pipérazinyl-1]-3 propyl]-3-3H-benzotriazine-1,2,3 one-4, chlorhydrate.

Obtenu en opérant comme dans l'exemple 2, à partir de 5,3 g (36 m. moles) de 3H-benzotriazine-1,2,3 one-4, de 9,7 g (36 m. moles) de (chloro-3 fluoro-4 phényl)-1 (chloro-3 propyl)-4 pipérazine, de 5 g (36 m. moles) de carbonate de potassium et de 100 $cm^3$ de N,N-diméthylformamide. Rdt : 8,4 g (53 %), F = 234-235 °C (éthanol — DMF).

Analyse centésimale $C_{20}H_{22}Cl_2FN_5O$ (M = 438,33)

Calculé : C 54,80 H 5,06 Cl 16,18 F 4,33 N 15,98 %
Trouvé : C 54,70 H 5,03 Cl 15,93 F 4,30 N 16,25 %.
I. R. $\bar{\nu}$ (C=O) = 1 680 $cm^{-1}$

La (chloro-3 fluoro-4 phényl)-1 (chloro-3 propyl)-4 pipérazine utilisée est obtenue comme dans l'exemple 13, à partir de 8,3 g (53 m. moles) de bromo-1 chloro-3 propane, de 9,4 g (44 m. moles) de (chloro-3 fluoro-4 phényl)-1 pipérazine [préparée selon B.W. Horrom et H.B. Wright, brevet américain 3.637.705 ; C.A. *76,* 113256a (1972)], de 7,3 g (53 m. moles) de carbonate de potassium et de 45 $cm^3$ de N,N-diméthylformamide. Rdt : 9,7 g (81 %), $Eb_{1,5}$ = 160-170 °C.

Exemple 28

Chloro-6 [(phényl-4 pipérazinyl-1)-3 propyl]-3-3H-benzotriazine-1,2,3 one-4, dichlorhydrate.

Obtenu en opérant comme dans l'exemple 1, à partir de 6 g (33 m. moles) de chloro-6-3H-benzotriazine-1,2,3 one-4, de 7,9 g (33 m. moles) de (chloro-3 propyl)-1 phényl-4 pipérazine, de 9,1 g (66 m. moles) de carbonate de potassium et de 165 $cm^3$ d'acétonitrile. Rdt : 6,8 g (45 %) F = 223-225 °C (éthanol).

Analyse centésimale : $C_{20}H_{24}Cl_3N_5O$ (M = 456,80)

Calculé : C 52,58 H 5,30 Cl 23,29 N 15,33 %
Trouvé : C 52,63 H 5,32 Cl 23,37 N 15,39 %.
I. R. : $\bar{\nu}$ (C=O) = 1 680 $cm^{-1}$

Exemple 29

[(Diméthoxy-3,4 phényl)-4 pipérazinyl-1]-3 propyl]-3-3H-benzotriazine-1,2,3 one-4, dichlorhydrate.

Un mélange de 6,9 g (47 m. moles) de 3H-benzotriazine-1,2,3 one-4, de 14 g (47 m. moles) de (chloro-3 propyl)-1 (diméthoxy-3,4 phényl)-4 pipérazine, de 6,5 g (47 m. moles) de carbonate de potassium et de 130 $cm^3$ de N,N-diméthylformamide est porté à 80 °C pendant 1 heure. Après refroidissement, les produits minéraux sont filtrés et le filtrat concentré à sec sous pression réduite. Le résidu est chromatographié sur colonne de silice (éluant acétate d'éthyle-méthanol 95 : 5). On obtient 4,7 g d'un solide fondant à 94-95 °C. Ce solide est dissout dans 250 $cm^3$ d'éthanol. A la solution obtenue on ajoute 2,6 $cm^3$ d'acide chlorhydrique 10 N et évapore à sec sous pression réduite. La recristallisation du résidu dans un mélange

éthanol-diméthylformamide fournit le dichlorhydrate du produit cherché. Rdt : 3,2 g (14 %). F = 204-206 °C.

Analyse centésimale : $C_{22}H_{29}Cl_2N_5O_3$ (M = 482,40)

Calculé : C 54,77 H 6,06 Cl 14,70 N 14,52 %
Trouvé : C 54,74 H 6,11 Cl 14,75 N 14,57 %.
I. R. $\bar{\nu}$ (C=O) = 1 695 $cm^{-1}$

La (chloro-3 propyl)-1 (diméthoxy-3,4 phényl)-4 pipérazine est préparée de la manière suivante : 11,5 g (70,2 m. moles) de bromo-1 chloro-3 propane sont ajoutés rapidement sous agitation à un mélange de 13 g (58,5 m. moles) de (diméthoxy-3,4 phényl)-1 pipérazine [préparée selon P.C. Jain et Coll. J. Med. Chem. *10,* 812 (1967)] et de 9,7 g (70,2 m. moles) de carbonate de potassium dans 60 $cm^3$ de N,N-diméthylformamide. L'agitation est poursuivie pendant 4 h 30 à température ambiante. Les produits minéraux sont alors filtrés et le filtrat conentré à sec sous pression réduite. Le résidu est dissous dans de l'éther et la solution obtenue lavée à l'eau puis séchée sur sulfate de sodium. Après évaporation de l'éther on obtient 14 g (Rdt : 80 %) de (chloro-3 propyl)-1 (diméthoxy-3,4 phényl)-4 pipérazine que l'on utilise sans autre purification.

## Exemple 30

[[(Chloro-3 phényl)-4 pipérazinyl-1]-3 propyl]-3 dichloro-6,8-3H-benzotriazine-1,2,3 one-4, chlorhydrate.

Obtenu en opérant comme dans l'exemple 29 ci-dessus à partir de 450 mg (2,1 m. moles) de dichloro-6,8-3H-benzotriazine-1,2,3 one-4 [préparée selon S. M. Gadekar et E. Ross J. Org. Chem. *26,* 613 (1961)] de 570 mg (2,1 m. moles) de (chloro-3 phényl)-1 (chloro-3 propyl)-4 pipérazine, de 290 mg (2,1 m. moles) de carbonate de potassium et de 20 $cm^3$ de N,N-diméthylformamide. Rdt : 0,15 g (15 %), F = 256-259 °C (éthanol-DMF).

Analyse centésimale : $C_{20}H_{21}Cl_4N_5O$ (M = 489,24)

Calculé : C 49,10 H 4,33 Cl 28,99 N 14,32 %
Trouvé : C 48,90 H 4,28 Cl 28,68 N 13,99 %
I. R. : $\bar{\nu}$ (C = O) = 1 685 $cm^{-1}$

## Exemple 31

[[(Chloro-3 phényl)-4 pipérazinyl-1]-2 éthyl]-3-3H-benzotriazine-1,2,3 one-4, chlorhydrate.

Un mélange de 10,5 g (50 m. moles) de (chloro-2 éthyl)3-3H-benzotriazine-1,2,3 one-4 [préparée selon K. Hasspacher et G. Ohnacker, brevet américain 3 316 262 C.A. *67,* 64445q )1967)] de 27 g (100 m. moles) de dichlorhydrate de (chloro-3 phényl)-1 pipérazine, de 42,3 g (300 m. moles) de carbonate de potassium, de 0,1 g d'iodure de potassium et de 150 $cm^3$ d'acétonitrile est porté à reflux pendant 40 heures. Le mélange est dilué avec 500 $cm^3$ d'eau et filtré. Le solide ainsi isolé est purifié par filtration sur silice (éluant acétate d'éthyle). On obtient la [[(chloro-3 phényl)-4 pipérazinyl-1]-2 éthyl]-3-3H-benzotriazine-1,2,3 one-4 fondant à 154-157 °C. Elle est dissoute dans de l'éthanol. A cette solution on ajoute 9,8 $cm^3$ d'aciode chlorhydrique 10 N et évapore à sec sous pression réduite. La recristallisation du résidu dans un mélange éthanol-DMF fournit le chlorhydrate cherché. Rdt : 11,5 g (57 %). F = 225-227 °C.

Analyse centésimale : $C_{19}H_{21}Cl_2N_5O$ (M = 406,31)

Calculé : C 56,16 H 5,21 Cl 17,45 N 17,24 %
Trouvé : C 55,97 H 5,45 Cl 17,32 N 17,01 %.
I. R. $\bar{\nu}$ (C = O) = 1 685 $cm^{-1}$

## Exemple 32

[[(Méthoxy-2 phényl)-4 pipérazinyl-1]-2 éthyl]-3-3H-benzotriazine-1,2,3 one-4, chlorhydrate.

Une solution de 7,7 g (40 m. moles) de (méthoxy-2 phényl)-1 pipérazine dans 20 $cm^3$ de N,N-diméthylformamide est ajoutée goutte à goutte à un mélange de 13,8 g (40 m. moles) de [(méthyl-4 benzènesulfonyl)-2 éthyl]-3-3H-benzotriazine-1,2,3 one-4 [préparée selon A. J. Barker et Coll. J. Chem. Soc. Perkin 1, *1979,* 2203] et de 6,1 g (44 m. moles) de carbonate de potassium dans 100 $cm^3$ de N,N-diméthylformamide. Le milieu réactionnel est agité 1 h à température ambiante puis porté 2 h 30 à 80 °C. Après refroidissement les produits minéraux sont éliminés par filtration et le filtrat concentré à sec sous

pression réduite. Le résidu est repris par un mélange d'eau et de chlorure de méthylène. La phase organique est lavée à l'eau, séchée sur sulfate de sodium et concentrée sous vide. Le résidu est dissous dans 250 $cm^3$ d'éthanol. A cette solution on ajoute 9,4 $cm^3$ d'acide chlorhydrique 10 N et évapore à sec sous pression réduite. Le chlorhydrate de [[(méthoxy-2 phényl)-4 pipérazinyl-1]-2 éthyl]-3-3H-benzotriazine-1,2,3 one-4 obtenu est purifié par recristallisation dans un mélange éthanol-DMF. Rdt : 8,7 g (54 %). F = 225-227 °C

Analyse centésimale $C_{20}H_{24}ClN_5O_2$ (M = 401,89)

Calculé : C 59,77 H 6,02 Cl 8,82 N 17,43 %
Trouvé : C 59,60 H 6,12 Cl 8,74 N 17,32 %.'
I. R. $\bar{\nu}$ (C = O) = 1 680 $cm^{-1}$

Exemple 33

[[(Chloro-3 phényl)-4 pipérazinyl-1]-4 butyl]-3-3H-benzotriazine-1,2,3 one-4.

a) [(Hydroxy-4 butyl)-1]-3-3H-benzotriazine-1,2,3 one-4

Une solution de 32,7 g (0,30 mole) de chloro-4 butanol-1 dans 235 $cm^3$ de N,N diméthylformamide est ajoutée goutte à goutte, à température ambiante, à un mélange de 27,7 g (0,188 mole) de 3H-benzotriazine-1,2,3 one-4 et de 28,6 g (0,207 mole) de carbonate de potassium dans 235 $cm^3$ de N,N-diméthylformamide. Le milieu réactionnel est porté à 80 °C pendant 3 heures. Les produits minéraux sont filtrés et le filtrat concentré à sec sous pression réduite. Le résidu est filtré sur silice (éluant : hexane-acétate d'éthyle 1 : 4). On obtient 24,2 g (Rdt : 59 %) d'[(hydroxy-4 butyl)-1]-3-3H-benzotriazine-1,2,3 one-4. F = 47-49,5 °C.
I. R. : $\bar{\nu}$ (C = O) = 1 665 $cm^{-1}$

b) [[(Méthyl-4 benzènesulfonyl)-4 butyl]-1]-3-3H-benzotriazine-1,2,3 one-4

On ajoute par portions 7,3 g (38 m. moles) de chlorure de méthyl-4 benzènesulfonyle à une solution refroidie à 0 °C de 4,2 g (18 m. moles) d)[(hydroxy-4 butyl)-1]-3-3H-benzotriazine-1,2,3 one-4 dans 90 $cm^3$ de pyridine. Le mélange est agité 4 h à 0 °C puis abandonné 4 jours à cette température. Il est alors jeté dans 750 $cm^3$ d'eau glacée. Le précipité est filtré, lavé à l'eau et séché. On obtient 4,9 g (Rdt 69 %) de [[(méthyl-4 benzènesulfonyl)-4 butyl]-1]-3-3H-benzotriazine-1,2,3 one-4 fondant à 80-84 °C et utilisé dans la suite sans autre purification.

c) [[(Chloro-3 phényl)-4 pipérazinyl-1]-4 butyl]-3-3H-benzotriazine-1,2,3 one-4

Un mélange de 17,5 g (46,9 m. moles) de [[(méthyl-4 benzènesulfonyl)-4 butyl]-1]-3-3H-benzotriazine-1,2,3 one-4, de 9,2 g (46,9 m. moles) de (chloro-3 phényl)-1 pipérazine de 7,1 g (51,4 m. moles) de carbonate de potassium et de 190 $cm^3$ de N,N-diméthylformamide est agité 7 h à température ambiante puis 3 h à 80 °C. Après refroidissement le milieu réactionnel est dilué avec 1 litre d'eau. Le précipité formé est filtré, lavé à l'eau et séché. Il est filtré sur colonne de silice (éluant : hexane-acétate d'éthyle 1 : 1) puis recristallisé dans un mélange hexane-acétate d'éthyle. On obtient ainsi 9,9 g (Rdt 53 %) de [[(chloro-3 phényl)-4 pipérazinyl-1]-4 butyl]-3-3H-benzotriazine-1,2,3 one-4. F = 107,5-109,5 °C.

Analyse centésimale : $C_{21}H_{24}ClN_5O$ (M = 397,90)

Calculé : C 63,39 H 6,08 Cl 8,91 N 17,60 %
Trouvé : C 63,38 H 6,12 Cl 8,78 N 17,50 %.
I. R. : $\bar{\nu}$ (C = O) = 1 685 $cm^{-1}$.

## Revendications

1. [(Phényl-4 pipérazinyl-1)-3 alcoyl]-3-3H-benzo- et thiénotriazine-1,2,3, ones-4 caractérisées par la formule

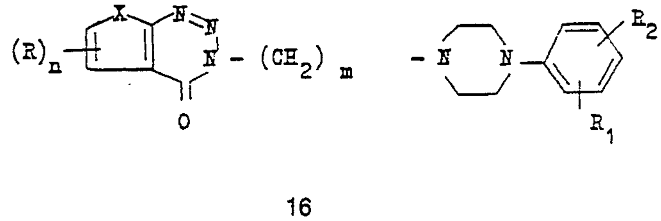

dans laquelle m est 2, 3 ou 4, X est le groupe vinylène —CH=CH— ou un atome de soufre ; $R_1$ et $R_2$ peuvent être égaux ou différents et sont l'hydrogène, un halogène, un radical alcoyle en $C_1$-$C_3$, alcoxy en $C_1$-$C_3$ ou trifluorométhyle ; lorsque X est —CH=CH— n est 0,1 ou 2 et R est l'hydrogène, un halogène, un radical alcoyle en $C_1$-$C_3$, alcoxy en $C_1$-$C_3$ ou le groupe nitro ; lorsque X est un atome de soufre, R est l'hydrogène ou constitue une chaîne $-(CH_2)_4-$ entre les deux positions libres du noyau thiophénique ; et leurs sels d'acides minéraux ou organiques pharmaceutiquement acceptables.

2. [(Phényl-4 pipérazinyl-1)-3 propyl]-3-3H-benzo- et thiénotriazine-1,2,3 ones-4 selon la revendication 1 caractérisées en ce que $R_1$ et $R_2$ peuvent être égaux ou différents et sont l'hydrogène ou un halogène ; et leurs sels d'acides minéraux ou organiques pharmaceutiquement acceptables.

3. [(Phényl-4 pipérazinyl-1)-3 propyl]-3-3H-benzo- et thiénotriazine-1,2,3 ones-4 selon la revendication 1 caractérisées en ce que $R_1$ est un atome de chlore situé en position méta par rapport à la pipérazine et $R_2$ est l'hydrogène ; et leurs sels d'acides minéraux ou organiques pharmaceutiquement acceptables.

4. Procédé de préparation des composés selon la revendication 1 caractérisé en ce qu'on traite une benzo- ou une thiéno-triazine-1,2,3 one-4 de formule

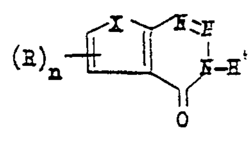

par un dérivé de formule

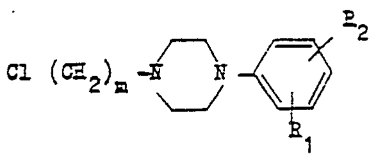

X, R, $R_1$, $R_2$ m et n ayant les significations données dans la revendication 1.

5. Procédé selon la revendication 4 caractérisé en ce qu'on effectue la réaction dans un solvant inerte en présence d'un agent alcalin, de préférence dans l'acétonitrile ou le N,N-diméthylformamide en présence d'un carbonate alcalin.

6. Procédé de préparation des composés selon la revendication 1 pour lesquels X et —CH=CH—, caractérisé en ce qu'on traite une benzotriazine-1,2,3 one-4 de formule

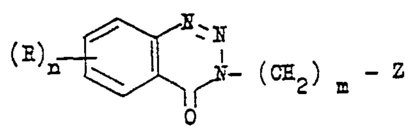

dans laquelle Z est un halogène, un groupe tosyle ou mésyle, par une phénylpipérazine de formule

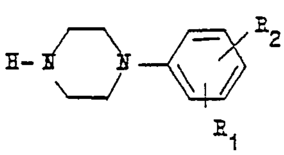

R, $R_1$, $R_2$, m et n ayant les significations données dans la revendication 1.

7. Procédé selon la revendication 6 caractérisé en ce qu'on effectue la réaction dans un solvant inerte en présence d'un agent alcalin ou d'un excès de la phénylpipérazine utilisée, de préférence dans l'acétonitrile, le N,N-diméthylformamide ou un alcanol de bas poids moléculaire en présence d'un carbonate alcalin.

8. Benzotriazine-1,2,3 ones-4 produits intermédiaires dans la préparation des composés selon la revendication 6 caractérisées par la formule

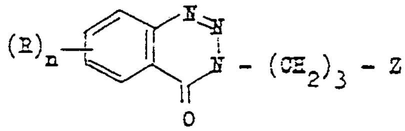

dans laquelle Z est un halogène, un groupe tosyle ou mésyle R et n ayant les significations données dans la revendication 1.

9. (Hydroxypropyl)-3 benzotriazine-1,2,3 ones-4 produits intermédiaires dans la préparation des composés selon la revendication 8 caractérisées par la formule

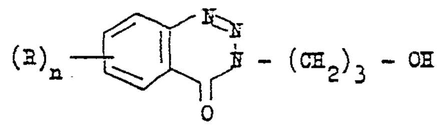

dans laquelle R et n ont les significations données dans la revendication 1.

10. Médicament contenant comme principe actif une [(phényl-4 pipérazinyl-1)-3 alcoyl]-3-3H-benzo- ou thiéno-triazine-1,2,3 one-4 selon la revendication 1 et leurs sels d'acides minéraux ou organiques pharmaceutiquement acceptables.

**Claims**

1. 3-[3-(4-phenyl-1-piperazinyl)-alkyl]-3H-benzo- and thieno-1,2,3-triazine-4-ones characterized by the formula

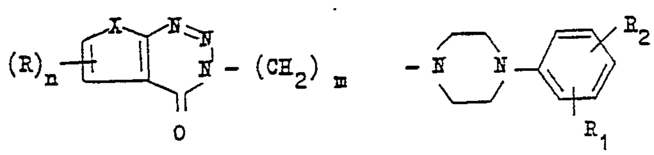

wherein m is 2, 3 or 4, X ist the vinylene group —CH=CH— or a sulfur atome ; $R_1$ and $R_2$ can be equal or different and are hydrogen, a halogen, a $C_1$-$C_3$ alkyl radical, $C_1$-$C_3$ alkoxy or trifluoromethyl ; if X is —CH=CH—, n is 0,1 or 2 and R is hydrogen, a halogen, a $C_1$-$C_3$ alkyl, radical, $C_1$-$C_3$ alkoxy or the nitro group ; if X is a sulfur atom, R is hydrogen or a $-(CH_2)_4-$ chain between the two free positions of the thiophene nucleus ; and the pharmaceutically acceptable salts of mineral acids or organic acids thereof.

2. 3-[3-(4-phenyl-1-piperazinyl)-propyl]-3H-benzo and thieno-1,2,3-triazine-4-ones according to claim 1, characterized in that $R_1$ and $R_2$ may be equal or different and are hydrogen or a halogen ; and the pharmaceutically acceptable salts of mineral acids or organic acids thereof.

3. 3-[3-(4-phenyl-1-piperazinyl)-propyl]-3H-benzo and thieno-1,2,3-triazine-4-ones according to claim 1, characterized in that $R_1$ is a chloro atome situated in the meta position relative to the piperazine and $R_2$ is hydrogen ; and the pharmaceutically acceptable salts of mineral acids or organic acids thereof.

4. Process of production of compounds according to claim 1, characterized in that a benzo or a thieno-1,2,3-triazine-4-one of the formula

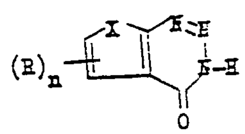

is treated with a derivative of the formula

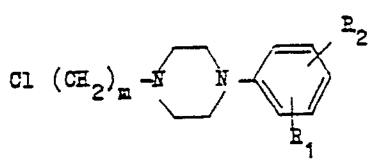

X, R, $R_1$, $R_2$ m and n having the meanings shown by claim 1.

5. Process according to claim 4, characterized in that the reaction is carried out in an inert solvent in presence of an alkaline agent, preferably in acetonitrile or N,N-dimethylformamide in presence of an alkali carbonate.

6. Process of preparation of compounds according to claim 1, X being —CH=CH—, characterized in that a 1,2,3-benzotriazine-4-one of the formula

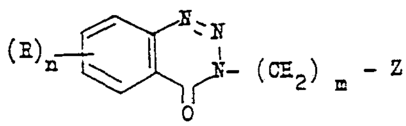

wherein Z is halogen or a tosyl or mesyl group, is treated with a phenylpiperazine of the formula

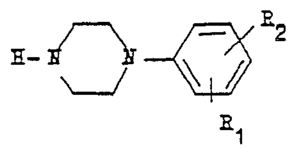

R, $R_1$, $R_2$, m and n having the meanings shown by claim 1.

7. Process according to claim 6, characterized in that the reaction is carried out in an inert solvent in presence of an alkaline agent or of an excess of the used phenylpiperazine, preferably in acetonitrile, N,N-dimethylformamide or an alkanol of low molecular weight in presence of an alkali carbonate.

8. 1,2,3-Benzotriazine-4-ones as intermediate products in the production of compounds according to claim 6, characterized by the formula

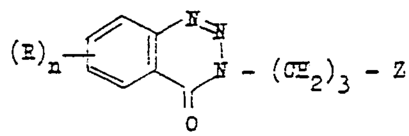

wherein Z is a halogen, a tosyl or mesyl group, R and n having the meanings shown by claim 1.

9. 3-(Hydroxypropyl)-1,2,3-benzotriazine-4-ones as intermediate products in the preparation of compounds according to claim 8, characterized by the formula

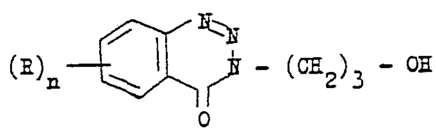

wherein R and n have the meanings shown by claim 1.

10. Drug containing as active principel a 3-[3-(4-phenyl-1-piperazinyl)-alkyl]-3H-benzo or thieno-1,2,3-triazine-4-one according to claim 1, and the pharmaceutically acceptable salts of mineral acids or organic acids thereof.

**Patentansprüche**

1. 3-[3-(4-Phenyl-1-piperazinyl)-alkyl]-3H-benzo- und -thieno-1,2,3-triazin-4-one, gekennzeichnet durch die Formel

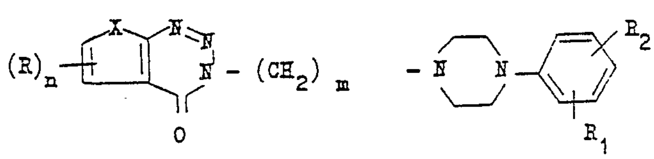

worin m 2, 3 oder 4 ist, X die Vinylengruppe —CH=CH— oder ein Schwefelatom ist, $R_1$ und $R_2$ gleich oder verschieden sein können und Wasserstoff, ein Halogen, einen $C_1$-$C_3$-Alkylrest, einen $C_1$-$C_3$-Alkoxyrest oder Trifluormethyl bedeuten, wenn X —CH=CH— ist, n 0, 1 oder 2 ist und R Wasserstoff, ein Halogen, ein $C_1$-$C_3$-Alkylrest, ein $C_1$-$C_3$-Alkoxyrest oder die Nitrogruppe bedeutet, und wenn X ein Schwefelatom ist, R Wasserstoff ist oder eine $-(CH_2)-_4$-Kette zwischen den beiden freien Positionen des Tiophenkerns bedeutet, und ihre pharmazeutisch verträglichen Salze von Mineralsäuren oder organischen Säuren.

2. 3-[3-(4-Phenyl-1-piperazinyl)-propyl]-3H-benzo- und -thieno-1,2,3-triazin-4-one nach Anspruch 1, dadurch gekennzeichnet, daß $R_1$ und $R_2$ gleich oder verschieden sein können und Wasserstoff oder ein Halogen bedeuten, und ihre pharmazeutisch verträglichen Salze von Mineralsäuren oder organischen Säuren.

3. 3-[3-(4-Phenyl-1-piperazinyl)-propyl]-3H-benzo- und -thieno-1,2,3-triazin-4-one nach Anspruch 1, dadurch gekennzeichnet, daß $R_1$ ein in der meta-Stellung bezüglich des Piperazins stehendes Chloratom ist und $R_2$ Wasserstoff ist, und ihre pharmazeutisch verträglichen Salze von Mineralsäuren oder organischen Säuren.

4. Verfahren zur Herstellung von Verbindungen nach Anspruch 1, dadurch gekennzeichnet, daß man ein Benzo- oder ein Thieno-1,2,3-triazin-4-on der Formel

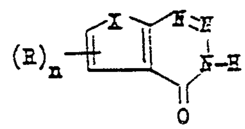

mit einem Derivat der Formel

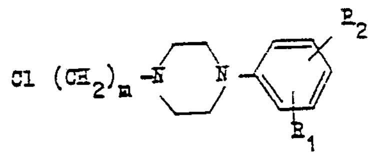

behandelt, worin X, R, $R_1$, $R_2$, m und n die im Anspruch 1 angegebenen Bedeutungen haben.

5. Verfahren nach Anspruch 4, dadurch gekennzeichnet, daß man die Umsetzung in einem inerten Lösungsmittel in Gegenwart eines alkalischen Mittels, vorzugsweise in Acetonitril oder N,N-Dimethylformamid in Gegenwart eines Alkalicarbonats, durchführt.

6. Verfahren zur Herstallung von Verbindungen nach Anspruch 1, worin X —CH=CH— ist, dadurch gekennzeichnet, daß man ein 1,2,3-Benzotriazin der Formel

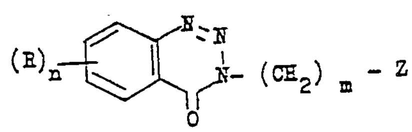

worin Z ein Halogen, eine Tosylgruppe oder Mesylgruppe bedeutet, mit einem Phenylpiperazin der Formel

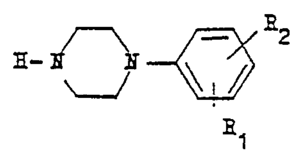

behandelt, wobei R, $R_1$, $R_2$, m und n die im Anspruch 1 angegebenen Bedeutungen haben.

7. Verfahren nach Anspruch 6, dadurch gekennzeichnet, daß man die Umsetzung in einem inerten Lösungsmittel in Gegenwart eines alkalischen Mittels oder eines Überschusses des benutzten Phenylpiperazins, vorzugsweise in Acetonitril, N,N-Dimethylformamid oder einem Alkanol mit niedrigem Molekulargewicht in Gegenwart eines Alkalicarbonats, durchführt.

8. 1,2,3-Benzotriazin-4-one als Zwischenprodukte bei der Herstellung von Verbindungen nach Anspruch 6, gekennzeichnet durch die Formel

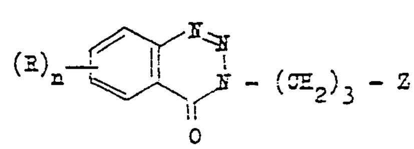

worin Z ein Halogen, eine Tosyl- oder Mesylgruppe ist und R und n die im Anspruch 1 angegebenen Bedeutungen haben.

9. 3-(Hydroxypropyl)-1,2,3-benzotriazin-4-one als Zwischenprodukte bei der Herstellung von Verbindungen nach Anspruch 8, gekennzeichnet durch die Formel

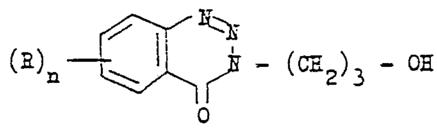

worin R und n die im Anspruch 1 angegebenen Bedeutungen haben.

10. Arzneimittel, enthaltend als Wirkstoff ein 3-[3-(4-Phenyl-1-piperazinyl)-alkyl]-3H-benzo- oder thieno-1,2,3-triazin-4-on nach Anspruch 1 und deren pharmazeutisch verträgliche Salze von Mineralsäuren oder organischen Säuren.